# EUROPEAN PATENT APPLICATION

(11) **EP 1 864 652 A1**
(43) Date of publication of application: **12.12.2007**
(21) Application number: 07252247.7
(22) Date of filing: 04.06.2007
(51) Int. Cl.: A61K 9/48, A61K 47/24, A61M 31/00

(54) **Personal lubricant compositions and kits for providing personal lubrication**

(30) Priority: 05.06.2006 US 803902 P; 01.05.2007 US 915279 P
(71) Applicant: McNeil-PPC, Inc., Skillman, NJ 08558 (US)
(72) Inventor: Gentner, Louis, Kinterville, PA (US); Joyce, Michael, Randolph, NJ 07869 (US); Lin, Shun Y., Plainsboro, NJ 08536 (US)
(74) Representative: Fisher, Adrian John

(57) **Abstract**

This invention relates to body orifice moisturizing compositions and kits and methods of their use. More particularly, it relates to vaginal moisturizing compositions and kits and methods of their use. The vaginal moisturizing compositions of this invention are preferably in the form of a soft gelatin capsule containing therein a lubricating fluid, said soft gelatin capsule being capable of insertion into a body orifice. It will provide sustained lubrication to the body orifice.

## Description

### FIELD OF THE INVENTION

The present invention relates to personal lubricant compositions, methods for providing personal lubrication during or prior to sexual intercourse, dosage forms for containing and delivering personal lubricant compositions and kits containing dosage forms and means for administering said dosage forms to the orifices of human beings.

### BACKGROUND OF THE INVENTION

Personal lubricants for intimate contact are well known. Typically, personal lubricants are marketed as liquids, jellies, gels or suppositories. Examples of such products include Replens®, K-Y® Jelly, Astroglide®, K-Y® Liquid, K-Y® Ultragel^{™}. More recently K-Y® Warming Liquid was introduced to the marketplace. K-Y ® Warming Liquid is a water soluble, anhydrous composition that warms on contact while providing lubrication.

This invention is directed to a personal lubricant and in particular to a vaginal lubricant, a novel dosage form and a method of delivering such vaginal lubricant.

A lubricating fluid is normally present in the vagina and during sexual arousal an increased amount of the fluid is produced. The lack of sufficient vaginal lubrication causes vaginal tissue to become dry and irritated and may result in painful sexual intercourse and sometimes bleeding. There are a number of causes for this condition including decreased estrogen levels during menopause or after surgical removal of ovaries and after radiation therapy.

Decreased sexual desire or arousal can also result in vaginal dryness. Oral contraceptives and certain medications such as antihistaminics, antidepressants, blood pressure, and cardiac medicines can contribute to vaginal dryness. Additionally, psychological conditions including stress, fatigue and anxiety may impede production of the natural lubricant. A combination of hormonal and psychological factors may induce dryness temporarily after childbirth particularly if the mother is breastfeeding.

Causes of vaginal dryness also include estrogen deficiency (menopause, oophorectomy, postpartum loss of placental estrogen, breastfeeding, radiation, chemotherapy, etc.), pharmacologic interventions (anticholinergics, antihistamines, etc.) and chemical sensitivities (douches, soaps, detergents, etc.). Vaginal dryness creates a variety of quality of life issues for women and their partners. Consequently, there is an unmet need by women who desire a vaginal moisturizer that is easy to use, less messy, long-lasting and can be inserted prior to intimacy to increase their spontaneity.

Common personal lubricants and vaginal moisturizers including K-Y Jelly and Vaseline are not ideal due to their messy and sticky consistency, poor lasting quality and lack of pleasant fragrance and acceptable taste. These lubricants and moisturizers must also be applied immediately prior to intercourse and do not allow for discreet insertion hours before. Many consumers are also unsure of the amount of product to use.

Vaginal lubricating suppositories are sold commercially, however, the available products are not preferred by consumers as they are somewhat inconvenient to use without an applicator in their current form.

Foam-type vaginal lubricants are also sold commercially. Some are packaged under pressure in containers similar to shaving cream cans.

Their use has poor acceptability because of the emission sound. Furthermore, they provide lubrication for only a relatively short time.

Most of the commercially available personal lubricant products are used by first applying to the hand or fingers of the user and then to the intimate area. This can be messy and, therefore, undesirable. Additionally, some individuals are adverse to directly applying a personal lubricant to the genital regions.

An optimal vaginal moisturizer should meet the following requirements:
- Non-hormonal treatment for relieving vaginal dryness
- Last for days
- Soothe and lubricate
- Non-irritating and non-sticky
- Fragrance free
- Discreet
- Convenient application
- Safe and gentle

Accordingly, we have developed a vaginal moisturizer product in a novel delivery form to meet the above-mentioned needs of the population of women who suffer from vaginal dryness.

### SUMMARY OF THE INVENTION

This invention is directed to compositions useful for personal lubrication, methods for providing personal lubrication during or prior to sexual intercourse, dosage forms for containing and delivering personal lubricant compositions and kits containing dosage forms and means for administering said dosage forms to the orifices of human beings. This invention further relates to a method for providing personal lubrication to a human body orifice involving the step of administering an appropriately measured single dose of an effective amount of personal lubricant in a pharmaceutically acceptable carrier to the interior of the human body orifice.

This invention is also directed to a kit for providing personal lubrication to a human body orifice containing: (a) a single dose of an effective amount of personal lubricant in a pharmaceutically acceptable carrier and in a form adapted to be administered within the human body orifice and (b) an applicator packaged with said single dose.

More particularly, this invention is directed to a composition and article of manufacture combining a lubricant material encased by a soft gelatin shell, wherein the lubricant material does not cause deterioration in the soft gelatin shell.

### DETAILED DESCRIPTION OF THE PREFERRED EMBODIMENT(S)

As used herein, the term "solid dosage form" means a dosage form that is applied intravaginally and does not flow perceptibly under moderate stress. Such solid dosage forms can take the form of a soft gelatin capsule or ovule containing lubricating ingredients in liquid, semisolid form or a suppository, which is defined as a small plug containing lubricating ingredients, said capsule or ovule being designed to melt at body temperature within a body cavity other than the mouth, especially the rectum or vagina.

The solid dosage form preferably may contain both water insoluble and water-soluble components. The water insoluble components may be any water insoluble components, which are acceptable for application to and not unduly irritating to the body cavity, including but not limited to stearyl alcohol, petrolatum, vegetable oil suppository bases or a combination thereof, preferably stearyl alcohol. Water soluble components may be any water soluble components, which are acceptable for application to and not unduly irritating to the body cavity, including but not limited to polyethylene glycols, propylene glycols and glycerin, as well as polysaccharide gums such as carboxymethylcellulose, xanthan gum, other carboxyalkylcellulose products and silica products. Said components may also include additional adjuvants that soothe or complement the vaginal or mucosal tissues such as vitamin A, vitamin C, vitamin E, humectants, analgesics, emollients, antiinflammatories and the like. Examples of additional fill mass compositions include those set forth in copending patent application Serial No. 11/224,870 filed September 13, 2005, which is hereby incorporated herein by reference.

Preferably, the fill mass compositions of this invention are made up of materials that are not water-soluble as water-soluble materials tend to cause deterioration in a soft gelatin shell. Preferably, the volume of the fill mass is between about 1 and about 4 cc, more preferably from about 1.5 to about 2.5 cc, and most preferably about 2.5 cc.

Solid dosage forms may be applied to the body cavity, and spread in the body cavity. At least part, and preferably all of, the dosage form is melted, preferably on contact with the body and most preferably from body heat. The dosage form is spread preferably substantially uniformly in the body cavity, preferably after the melting of the dosage form has occurred. The dosage form used may be, including but not limited to, any embodiment described above. Another embodiment of the invention comprises a method of delivering a personal lubricant composition (for example those described in the other embodiments) into a body cavity, including the vaginal cavity or the rectum in a gelatin capsule with or without an applicator. Such gelatin capsules are known in the art and are used in connection with products such as Monistat® 1 combination pack (by McNeil-PPC, Inc., Skillman, New Jersey). The gelatin capsule may comprise a soft gelatin capsule shell or a two-piece hard gelatin capsule shell, preferably a soft gelatin capsule shell. The shell encases the personal lubricant of this invention (as claimed and taught herein).

Yet other embodiments of this invention include compositions useful as vaginal moisturizers. Such compositions must be compatible with soft gelatin capsules. For example, they should be moisturizing but should not include aqueous elements so as not to erode the gelatin capsule enclosures of the dosage forms of this invention. Vaginal moisturizer ingredients may include silicone oils, Cyclomethicone, Sorbitol, Ammonium Alginate, Xylitol, Aluminum Hydroxide Adjuvant, Triethanolamine, Trehalose, Polydextrose, Cyclodextrin and the like.

More preferably, such materials include one or more silicone fluids, such as dimethicone and dimethiconol. Most preferably, the fill mass is a silicone fluid such as that currently sold commercially as K-Y® Intrigue Premium Personal Lubricant, available from Personal Products Company, Skillman, NJ.

Preferably, the viscosity of the fill mass of the article of manufacture of this invention is from about 100 to about 500 cps. More preferably, the viscosity is from about 100 to about 200 cps. Most preferably, the viscosity is about 100 cps. Viscosity may be adjusted utilizing appropriate combinations of silicone materials contained therein such as dimethicone and dimethiconol having varying chain lengths.

Applicators appropriate for use with the capsule or ovule of this invention include those described in U.S. Patent No. 7,104,968, and copending patent applications Serial Nos. 10/366,710, filed February 12, 2003 and 10/806,947, filed March 23, 2004 which are hereby incorporated herein by reference, or other suitable applicators known to those of skill in the art.

In another embodiment of the compositions and articles of manufacture of this invention, a solid suppository dosage fill mass containing hard fat such as hydrogenated vegetable fat and other lubricant ingredients may be encased within a soft gelatin capsule shell as set forth above, containing gelatin and glycerin. The solid suppository fill mass is preferably an anhydrous composition composed of a hard fat material that melts at or about body temperature (approximately 35°C to about 39°C), and can include hard fats such as hydrogenated vegetable oils such as Wecobee FS or Wecobee M (available commercially from The Stepan Company of Northfield, Illinois) or a combination thereof. Preferably, the fill mass is anhydrous and the hydrogenated vegetable fat comprises the remainder of the fill mass in addition to the active ingredient. Preferably, the weight ratio of the combination of fats is approximately 1:1 to about 2:1. More preferably, if a combination of hard fats is utilized, Wecobee M constitutes the minor proportion of the hard fat portion of the composition.

Further, this embodiment may utilize bioadhesive agents which help to promote adhesion of the ointment to the body cavity mucosa membranes. The bioadhesive agents (including polymers such as polycarbophil, gelling agents and hydrocolloids) may be any bioadhesive agent which is acceptable for application to and not unduly irritating to the body cavity, preferably xanthan gum, sodium carboxymethylcellulose, or mixtures thereof, most preferably a mixture of xanthan gum and sodium carboxymethylcellulose. The bioadhesive agents allow the dosage form to be applied and melted in the vagina, where the dosage form comes into contact with moisture. Then, elements within the dosage form gel and therefore the lubricant is retained for sufficient time to effectively lubricate the vagina.

Additionally, this embodiment may include one or more dispersing agents, which may be any dispersing agents acceptable for application to and not unduly irritating to the body cavity, preferably silicon dioxide. Dispersing agents contribute homogenous melt and spread characteristics to the mixture and aids in the adhesion to the body cavity mucous membrane for a controlled release of the lubricant.

The water insoluble components may be any water insoluble components, which are acceptable for application to and not unduly irritating to the body cavity, including but not limited to petrolatum, vegetable oil bases or a combination thereof. The water soluble components may be any water soluble components, which are acceptable for application to and not unduly irritating to the body cavity, including but not limited to polyethylene glycols, propylene glycols and glycerin.

The combination of water soluble and water insoluble components are utilized for the base. The water soluble components preferably include components with a mixture of both high and low melting points, more preferably polyethylene glycols, propylene glycols and glycerin. Most preferably, polyethylene glycol 400, which is a liquid, is combined with polyethylene 3350, which is a solid. Additionally, the water insoluble components preferably include components with a mixture of both high and low melting points, more preferably petrolatum and vegetable oils with both high and low melting points.

We anticipate that the soft gelatin capsule of the dosage form of this invention will, in use, be less messy than currently-available liquid and suppository lubricants and may be designed to afford time-release or sufficient delay between insertion, melting of the capsule and full lubrication to make it more convenient and longer lasting than currently available suppository lubricants. The soft gelatin capsule of the dosage form of this invention should also be easier to carry and more discreet than other lubricant forms such as bottled liquid lubricant or tubes of jelly lubricant.

The soft gelatin capsule dosage form of this invention may be applied to a body cavity discreetly prior to sexual intercourse such that a sexual partner may not need to become aware of the fact that it has been used. The soft gelatin capsule dosage form of this invention is preferably in the form of an "ovule", similar in shape and appearance to that of the soft gelatin capsules sold commercially in the Monistat® 1 combination pack (by McNeil-PPC, Inc., Skillman, New Jersey). The ovule composition should allow the application to be discreet and less messy while providing long-lasting, lubrication prior to and during intimacy. It will permit partners to be more spontaneous in their intimacy and allow women to feel confident that they will not have vaginal dryness, thus permitting them to be more spontaneous. Because the ovule can be inserted prior to intimate acts, it will allow for uninterrupted intimacy, eliminating the need to reapply during the act. The ovule composition should be easy to use and provide long-lasting relief and comfort from vaginal dryness and, in effect, restore vaginal moisture levels. It may be used by women who are perimenopausal and post menopausal, who may be suffering from vaginal dryness, to ease the discomfort associated with dryness. Women wanting to enhance intimate moments with their partner by use of a personal lubricant may also want to use this product.

This invention also relates to methods of treatment and use of a vaginal moisturizer according to this invention in permitting women to be more spontaneous with intimacy. It may be useful in making intercourse more comfortable and enhances intimacy between a woman and her partner. It may also be useful in making sexual experience more pleasurable and is an discreet product to use. The products of this invention may be useful in providing moisturization that women perceive to feel like their own natural moisture and to relieve vaginal dryness. Preferably, the vaginal moisturizing products of this invention provide moisturization for at least three and more preferably four days without re-insertion or administering an additional product to the vagina or other body orifice in which it is used.

### Example

A monadic, home use consumer test was conducted using products of this invention. Approximately 600 women with what they characterized as "bothersome" vaginal dryness due to menopause who used personal lubricants or vaginal moisturizers on a regular basis were enrolled into each study group. All women were in committed relationships (married/living together for at least one year) and engaged in intercourse with their partner at least once every two weeks.

Each woman was given one of four silicone-filled soft gelatin products or one of either K-Y® Long-Lasting Vaginal Moisturizer (available commercially from Personal Products Company, Skillman, NJ) or Replens® vaginal moisturizer (available commercially from L'il Drug Store Products, Cedar Rapids, IA) (Product 2), which she was to use in her home over a 14-day test period. The products of this invention included a low-volume (1 cc), low viscosity product; a low-volume, high viscosity product; a high-volume (2.5 cc), low viscosity product (Product 1) and a high-volume, high viscosity product. Each woman was asked to engage in intercourse three times according to the following predetermined schedule:
(1) Insert a first product in the morning with an applicator (Day 1) and have sex on Day 3
(2) Wait at least three more days before using the second product (Day 6)
(3) Insert second product in the morning with an applicator and have sex that night
(4) Do not use third product until after completion of an online questionnaire
(5) Insert the third product in the morning without an applicator and have sex that night.
(6) Questionnaire results were summarized by percent (%) of subjects reporting the top two responses (top two boxes - "T2B").

In the resulting study, there was high acceptance for all four size/viscosity combinations of this invention that were evaluated. The 2.5cc low viscosity combination, however, trended to receive the highest scores. These results are reflected in Table 1 below.

**Table 1:**

| | 1cc High Viscosity | 1cc Low Viscosity | 2.5cc High Viscosity | 2.5cc Low Viscosity |
|---|---|---|---|---|
| Base Size | 90 | 86 | 75 | 79 |
| Allows me to be more spontaneous with intimacy | 79% | 81% | 79% | 84% |
| Is a discreet product to use | 90% | 95% | 93% | 91% |
| Makes intercourse more comfortable | 83% | 86% | 87% | 89% |
| Enhances intimacy between me and my partner | 78% | 77% | 76% | 82% |
| Makes sexual experience more pleasurable | 79% | 84% | 81% | 87% |
| Is an innovative product | 88% | 85% | 85% | 90% |
| Helps me feel confident | 69% | 70% | 75% | 77% |
| Helps me feel youthful | 51% | 62% | 61% | 68% |

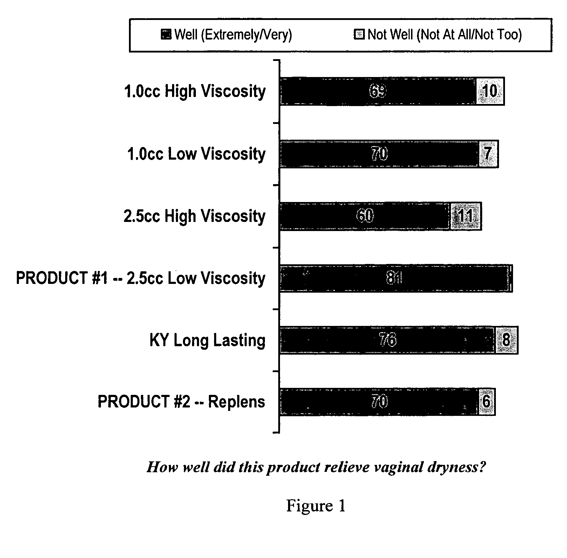

Regarding dryness relief specifically, each combination provided relief with the 2.5cc low viscosity product performing the best. In response to the question, *"How well did this product relieve vaginal dryness?"* the resulting response is set forth in Figure 1. This Figure indicates that the low viscosity, high volume product performed well and better than Product 2.

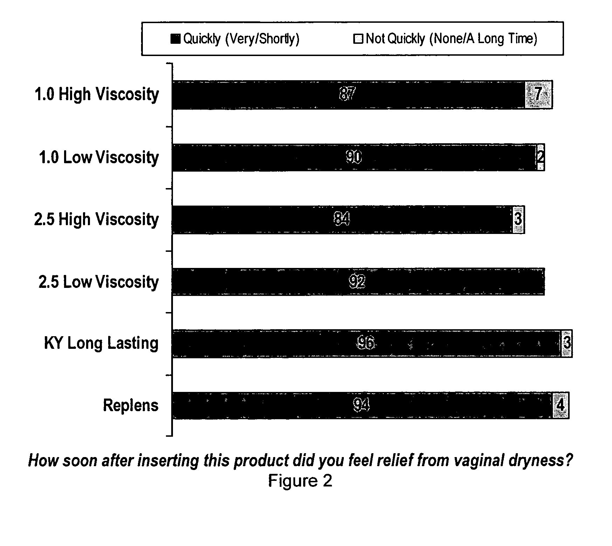

All products of this invention were rated very highly for quickness of relief from vaginal dryness, as shown in Figure 2.

After inserting the product of this invention on Day 1, subjects were asked, *"How would you describe the feeling of moisturization you experienced?"* for Days 1 through 6. Moisturization was measured using a moisturization level scale, as follows: 3=very moisturized, 2=slightly moisturized, 1=not at all moisturized. The mean moisturization scores for all the product prototypes were then calculated. All four product prototypes received mean moisturization scores greater than 2 through Day 3. However only the 1.0cc high viscosity product and the 2.5cc low viscosity product received mean moisturization scores greater than 2 for Day 4 (2.06 & 2.11 respectively). All mean moisturization scores were less than 2 for Days 5 & 6. Mean moisturization scores were slightly higher after the second and third product insertions, but all scores were still less than or equal to 2 by Day 5.

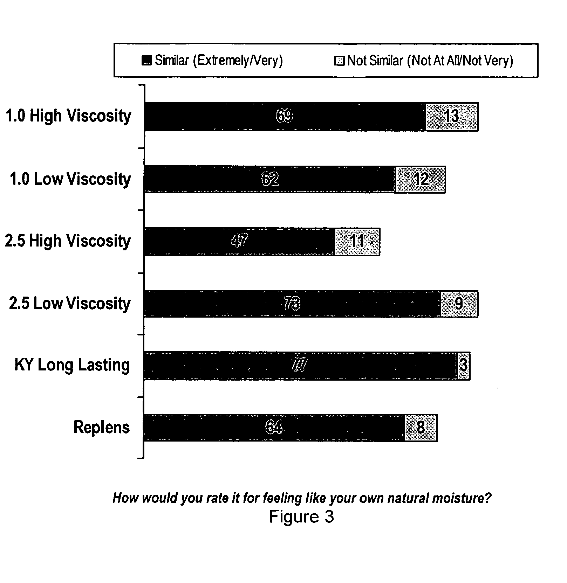

The 2.5cc low viscosity product had the highest T2B for the question, *"How would you rate it for feeling like your own natural moisture?"* as set forth in Figure 3.

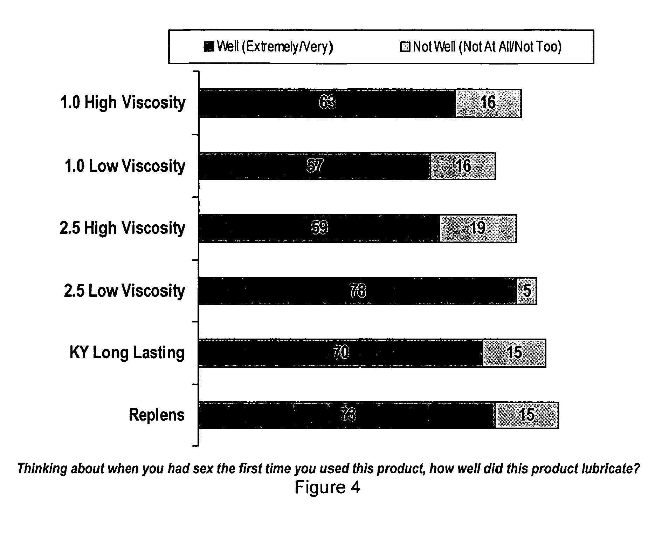

The 2.5cc low viscosity product had the highest T2B and lowest B2B for the question, *"Thinking* about when *you had* sex *the first* time *you used this product, how well did this product lubricate?".* These results are illustrated in Figure 4.

Users of the 2.5cc low viscosity product showed the strongest preference for insertion with an applicator (70%) compared to the 1.0 high viscosity product users (49%).

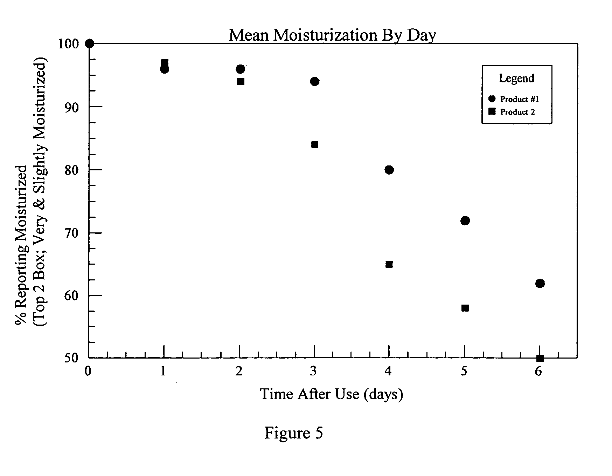

Further, in comparing the results of Product 1 of this invention and Product 2, product commercially available, the 2.5cc low viscosity product of this invention produced a higher percentage of moisturization that fell within the "extremely" and "very well" categories of response. The 2.5cc low viscosity product of this invention resulted in a sustained level of moisturization over a four-day period. These results are illustrated in Figure 5.

All four product size/viscosity combinations were reported to:
- quickly relieve vaginal dryness
- maintain vaginal moisture for days following a single use
- be similar to natural vaginal moisture
- provide effective lubrication for intimacy
- improve spontaneity for intimacy.

The 2.5cc low viscosity product combination tended to out perform the other combinations and provided relief of vaginal dryness for up to four days following a single use and should be marketed.

Users of the 2.5cc low viscosity product preferred an applicator.

Based on the results of this consumer study, the products of this invention were well accepted and fulfill the requirements of an optimal non-hormonal vaginal dryness relief product.

It will be understood by person by persons skilled in the art that various changes in the details, components, steps, and arrangements of the components and steps which have been described and illustrated in order to explain the nature of this invention may be made by those skilled in the art without departing from the principle and scope of the invention as expressed in the following claims.

## Claims

1. A personal lubricant dosage form comprising a soft gelatin capsule shell and a fill mass comprising a lubricant composition selected from the group consisting of a silicone and an anhydrous lubricant composition, said lubricant composition being insoluble with the outer capsule shell.

2. A kit for providing personal lubrication consisting essentially of:
(a) a single dose of a personal lubricant comprising a lubricant composition selected from the group consisting of a silicone and an anhydrous lubricant in a pharmaceutically acceptable carrier and in a form adapted to be administered intra-vaginally comprising a soft gelatin capsule surrounding said dose of personal lubricant; and
(b) an applicator adapted to apply said dose to a body cavity.

3. A method of providing a lubricant to a body cavity comprising placing a single dose of a personal lubricant comprising a lubricant composition selected from the group consisting of a silicone and an anhydrous lubricant in a pharmaceutically acceptable carrier and in a form adapted to be administered intra-vaginally comprising a soft gelatin capsule surrounding said dose of personal lubricant in an applicator adapted to apply said dose to a body cavity and applying said dose to a body cavity prior to sexual intercourse or when needed for relief of vaginal dryness.

4. A method of increasing moisturization in an individual's vagina comprising administering to an individual's vagina the composition of claim 1.

5. A method according to claim 4 wherein the level of said moisturization is sustained for at least four days.

6. A personal lubricant dosage form according to claim 1, wherein said fill mass has a volume of from about 1 cc to about 4 cc.

7. A personal lubricant dosage form according to claim 1, wherein said fill mass has a volume of from about 1.5 cc to about 2.5 cc.

8. A personal lubricant dosage form according to claim 1, wherein said fill mass has a volume of about 2.5 cc.

9. A personal lubricant dosage form according to claim 1, wherein said silicone is selected from the group consisting of dimethicone, dimethiconol and combinations thereof.

10. A personal lubricant dosage form according to claim 1, wherein said fill mass has a viscosity ranging from about 100 cps to about 500 cps.

11. A personal lubricant dosage form according to claim 1, wherein said fill mass has a viscosity ranging from about 100 cps to about 200 cps.

12. A personal lubricant dosage form according to claim 1, wherein said fill mass has a viscosity of about 100 cps.

13. A method according to claim 4 wherein said moisturization is sustained for at least three days.

14. A method according to claim 4 wherein said moisturization is sustained for at least two days.

15. A method according to claim 4 wherein said moisturization is sustained for at least one day.

16. A method according to claim 5, wherein said moisturization is sustained for at least four days at a mean moisturization level of at least 2.

17. A kit for enhancing intimacy between a woman and her partner consisting essentially of:
(a) a single dose of a personal lubricant comprising a silicone or anhydrous lubricant composition in a pharmaceutically acceptable carrier and in a form adapted to be administered intra-vaginally comprising a soft gelatin capsule surrounding said dose of personal lubricant; and
(b) an applicator adapted to apply said dose to a body cavity.
